# EUROPÄISCHE PATENTSCHRIFT

(11) **EP 2 643 031 B1**
(45) Veröffentlichungstag und Bekanntmachung des Hinweises auf die Patenterteilung: **24.06.2015**
(21) Anmeldenummer: 11788061.7
(22) Anmeldetag: 24.11.2011
(51) Int. Cl.: A61L 29/16, A61L 31/16

(54) **IMPLANT ZUR BEHANDLUNG ODER PRÄVENTION EINES ANEURYSMAS**
IMPLANT FOR TREATING OR PREVENTING ANEURISM
IMPLANT POUR LE TRAITEMENT OU LA PRÉVENTION D'UN ANÉVRISME

(30) Priorität: 26.11.2010 DE 202010015929 U; 24.02.2011 DE 102011012224; 14.07.2011 DE 102011107152
(43) Veröffentlichungstag der Anmeldung: 02.10.2013
(73) Patentinhaber: Ghotbi, Reza, 80689 München (DE)
(72) Erfinder: Ghotbi, Reza, 80689 München (DE)
(74) Vertreter: Lahrtz, Fritz
(86) Internationale Anmeldenummer: PCT/EP2011/005918
(87) Internationale Veröffentlichungsnummer: WO 2012/069193

(56) Entgegenhaltungen:
- WO-A2-2008/067288
- US-A1- 2005 154 451
- BRIAN C. CAPELL: "A farnesyltransferase inhibitor prevents both the ontset and late progresion of cardiovascular disease in a progeria mouse model", PROC NATL ACAD SCI USA, Bd. 105, Nr. 41, 14. Oktober 2008 (2008-10-14), Seiten 15902-15907, XP002669523,

## Beschreibung

### Gebiet der Erfindung

Die Erfindung betrifft ein medizinisches Produkt, das in ein Blutgefäß einführbar oder implantierbar ist, und das mindestens ein Medikament enthält.

### Hintergrund der Erfindung

Aneurysmen sind permanente Erweiterungen des Querschnitts von Blutgefäßen. Aneurysmen treten relativ häufig auf. Etwa 1 bis 5 % der mitteleuropäischen Bevölkerung sind Träger von Aneurysmen. Meist treten Aneurysmen lokal auf. Es sind jedoch auch zahlreiche Fälle bekannt, in denen Aneurysmen multipel an verschiedenen Körperstellen auftreten. Das multiple Auftreten von Aneurysmen wird auch als *Morbus aneurysmaticus* bezeichnet.

Je nach Größe des Aneurysmas und/oder der Lokalisierung desselben im Körper des Patienten kann das Auftreten eines Aneurysmas für den Patienten lebensbedrohlich sein oder zumindest starke Symptome hervorrufen. So kann ein Aneurysma beispielsweise mechanischen Druck auf einen Nerv ausüben und somit zu Lähmungen führen oder, wenn es zu einer Ruptur (Reißen oder Platzen) des Aneurysmas kommt, der Patient innerlich verbluten. Alternativ kann eine Ruptur zunächst auch zu einem pulsierenden Hämatom führen, das im günstigen Fall innerhalb mehrerer Wochen durch eine Bindegewebskapsel umgeben wird. In den meisten Fällen kommt es jedoch auch nach der vorübergehenden Ausbildung eines pulsierenden Hämatoms im weiteren Verlauf zu einer starken inneren Blutung, die nicht selten zum Verbluten des Patienten führt.

Ferner kann ein Aneurysma weitere Fehlfunktionen des Körpers verursachen. So kann es beispielsweise zu einer Nierenkolik, zu einer Embolie oder zu einer Thrombose führen und einen Schlaganfall oder Herzinfarkt auslösen. Grundsätzlich können Fehlfunktionen des Körpers dadurch hervorgerufen werden, dass das Aneurysma anschwillt und dadurch mechanischen Druck auf das umliegende Gewebe ausübt oder das Lumen von Gefäßen, wie Blut- oder Lymphgefäßen verringert, zu Blutungen führen und Blutgerinnsel verursachen, die ihrerseits das Lumen von Gefäßen, wie Blut- oder Lymphgefäßen verringern. Dem Fachmann sind zahlreiche Folgeschäden von Blutungen und verminderter Durchblutung, vermindertem Lymphfluss und mechanischen Nervenreizungen bekannt.

Die Gefahr, die von einem Aneurysma ausgeht hängt in der Regel von dessen Größe und der Lokalisation desselben ab, wobei insbesondere die Ruptur eines intracranialen Aneurysmas oder eines Aortenaneurysmas häufig für den Patienten tödlich enden. Eine Ruptur eines intracranialen Aneurysmas kann zur Hirnblutung (z.B. Subarachnoidalblutung), eine Ruptur eines Aortenaneurysmas beispielsweise zu einem intrapernetonealen Hämatoms (Hämaskos) führen.

Klinisch wird zwischen symptomatischen, asymptomatischen und bereits ruptierten Aneurysmen unterschieden. Gerade asymptomatische Aneurysmen werden häufig erst spät als Zufallsfund erkannt. Typischerweise wird die Diagnose eines Aneurysmas heute durch Betrachtung (Inspektion), Ertastung (Palpation), Ultraschall-Untersuchung(en) (Sonographie(n)), Röntgendiagnostik, Computertomographie (CT), Angiographie oder Magnetresonanztomographie (MRT) vorgenommen.

Auf Grund der Gefahr, die von einem Aneurysma ausgeht, werden heutzutage zumindest größere Aneurysmen präventiv behandelt. Der Grenzwert, wann ein Aneurysma als behandlungsbedürftig angesehen wird, hängt von zahlreichen Einzelfaktoren ab, de dem medizinischen Fachpersonal vorbehalten bleiben, zumeist werden Aneurysmen jedoch zumindest dann behandelt, wenn der Durchmesser etwa 50 % gegenüber dem Durchmesser des gesunden Gefäßes vergrößert ist. Die Behandlung kann grundsätzlich chirurgisch oder endovaskulär erfolgen.

Die chirurgische Behandlung setzt voraus, dass das Gefäß zunächst freigelegt wird. Eine solche Freilegung ist insbesondere bei intracranialen Aneurysmen äußerst riskant, da eine Kraniotomie (offene Hirn-Operation) vorgenommen werden muss. Aber auch Operationen an der freigelegten Aorta sind auf Grund der großen Blutmenge durchaus lebensgefährlich. Wenn das Gefäß entsprechend freigelegt worden ist, wird der Aneurysmasack meist mittels einer Klammer abgeklemmt (Clipping) und das Aneurysma so vom Blutkreislauf getrennt. Alternativ oder zusätzlich kann die Aneurysmawand auch von außen wandverstärkt werden (Wrapping). Da jedoch eine solche chirurgische Behandlung aufwändig, kostenintensiv und gefährlich ist, wird heutzutage zunehmend bevorzugt, Aneurysmen endovaskulär zu behandeln.

Bei der endovaskulären Behandlung von Aneurysmen wird in der Regel ein hohler Mikrokatheter über ein gut zugängliches Gefäß, wie beispielsweise die Leistenarterie, durch das Gefäßsystem zu der aneurysmatischen Stelle geführt und dort sogenannte Coils in den Aneurysmasack eingebracht. Hierbei wird meist ausgenutzt, dass die Oberflächen der Coils als körperfremde Oberfläche eine lokale Thrombenbildung verursachen und somit die Blutzirkulation im Aneurysma verhindert. Alternativ kann ein Gefäß auch endovaskulär durch eine Gefäßstütze (Stent) verstärkt werden.

Die Verfahren, die im Stand der Technik bekannt sind haben jedoch allgemein den Nachteil, dass durch sie nur die Symptome eines bereits aufgetretenen Aneurysmas behandelt werden, jedoch nicht die Ursache der Bildung eines Aneurysmas behandelt wird, um Folgeerkrankungen, wie das Auftreten weiterer Aneurysmen zu verhindern.

Nach einer endovaskulären Ausschaltung eines Aneurysmas mit einem Endograft kann insbesondere an dem sogenannten "Aneurysmahals" eine Erweiterung auftreten, die zur Fehlfunktion der Rekonstruktion führt. Der langfristiger Erfolg der endovaskulären Therapie ist wesentlich durch dieses Problem limitiert.

Hervorgerufen werden Aneurysmen durch zahlreiche Ursachen, wie beispielsweise degenerative Gefäßerkrankungen (z.B. Arteriosklerose), Traumata, Infektionen (z.B. rheumatisches Fieber, Syphilis, Lyme-Borreliose), Entzündungen, angeborene Biridegewebsschwächen, eine Schwäche der Endothelzellen, Marfan-Syndrom, Ehlers-Danlos-Syndrom, Herzfehler (z.B. Rechts-Links-Shunt), das Auftreten des Kawasaki-Syndroms, durch äußere Verletzungen und/oder durch innere Verletzungen. Besonders häufig werden Aneurysmen durch Arteriosklerose hervorgerufen.

Atherosklerose oder Arteriosklerose, die eine Krankheit basiert auf Gefäßalterungsprozesse ist, kann durch verschiedene Risikofaktoren wie Rauchen, cholesterinreiche Ernährung oder Zuckerkrankheit beschleunigt werden und zu Ablagerungen in den Arterien, Plaques, führen. Beim Erreichen einer bestimmten Größe, entsteht eine Stenose, Verengung der Arterie. In diesem Fall wird die Durchblutung der Organe vermindert. Solche Verengungen können neben der Ballonaufdehnung auch mittels Stentimplantation oder Bypässe behandelt werden. Die Implantate werden in die Arterie eingesetzt, um die Arterie offen zu halten.

Zur Behandlung der Arteriosklerose wird in der Gefäßmedizin von Implantaten Gebrauch gemacht, in dem der Therapeut die verengte Stelle des Blutgefäßes mit einem Implantat (z.B. einem Stent oder Stentgraft) versieht, sie aufdehnt (Ballonangioplastie, Drug eluted Ballon) oder er überbrückt die verengte Stelle mit einem Bypass aus Kunststoff oder körpereigenem Material.

Bei einer medizinischen Behandlung, Perkutane Transluminale Angioplastie, PTA, auch als Ballondilatation bekannt, wird die Stenose, die die Arterie verstopft, mit einem dünnen Katheter, der in der Regel aus Kunststoff beschaffen ist und dessen Ende ein aufblasbares Teil (Ballon) enthält, beseitigt. Eingeführt wird dieser Katheter meist über ein Gefäß in der Leiste. Von dort aus wird er unter Beobachtung auf einem Bildschirm in das betroffene Gefäß hineingeführt. Ist der Katheter in der Stenose angekommen, wird der Ballon aufgepumpt und drückt dadurch den Plaque an die Gefäßwand. Diese Anwendung ist auch als Ballondilatation bekannt. Nach der Entfernung des Ballons kann das Blut wieder durch das Gefäß fließen. Das betroffene Organ wird wieder mit Blut versorgt.

Bei vielen Patienten verschließt sich nach einer Ballondilatation die eröffnete Arterie innerhalb einiger Monate wieder. Damit das Gefäß länger offenbleibt, implantiert der Arzt nach einer erfolgreichen Ballondilatation in besonderen Fällen ein Stent. Dieser Eingriff wird als Stentimplantation bezeichnet. Teilweise werden Stents eingesetzt, die medikamentöse Substanzen in die Gefäßwand abgeben, welche zusätzlich einen Wiederverschluss verhindern sollen. Dadurch kann in vielen Fällen eine Restenose (Wiederverschluss) verhindert oder zeitlich hinausgeschoben werden.

Ist aus technischen oder morphologischen Gründen eine endovaskuläre Maßnahme wenig Erfolg versprechend, wird mit einem Bypass aus Kunststoff oder körpereigener Vene die Stenose überbrückt.

Die vaskuläre bzw. endovaskuläre Applikation von Medikamenten zur Therapie der Arteriosklerose mittels (Drug eluted Ballons and Stents engl.), mit Medikamenten beschichteten Prothesen bzw. Endoprothesen, findet ihre Anwendung als Stand der Technik.

Aus EP2193813A2 ist eine örtliche Behandlung der Blutgefäßerkrankungen mit Medikamenten beschichteten Implantaten bekannt, die beispielsweise eine Restenose verlangsamen oder gar verhindern sollen. Jedoch sind auch hier wie bei der Behandlung eines Aneurysmas die erzielten Ergebnisse in der Praxis nicht zufriedenstellend.

Weiterhin ist bekannt, dass das Verfahren aus EP2193813A2 für die Behandlung der Arteriosklerose unter Gebrauch von einem Krebsmedikament angewandt wird (Tepe G, Schmitmeier S, Speck U, Schnorr B, Kelsch B, Scheller B., The Journal of Cardiovascular Surgery, Feb. 2010).

Die orale Applikation von Medikamenten ruft jedoch zahlreiche unerwünschte Nebenwirkungen hervor, die in der Therapie nicht vertretbar sind.

Alternativ oder zusätzlich zu dem Hervorrufen von Aneurysmen durch Arteriosklerose können Aneurysmen auch durch eine Degeneration der Gefäßwände, insbesondere am Aneurysmahals hervorgerufen werden.

Diese Degeneration der Gefäßwände ist mit zunehmendem Alter in der Regel fortschreitend, insbesondere bei Menschen mit Gefäßerkrankungen, wie beispielsweise dem Marfan-Syndrom. Ferner kann eine Degeneration der Gefäßwände durch ein vermehrtes Aufkommen von Proteasen und einen zunehmenden Untergang von glatten Muskelzellen in der Muskularisschicht hervorgerufen werden. Gerade bei Patienten mit einer konsekutiven Dilatation kann das Ergebnis nach endovaskulärer Therapie deutlich limitiert sein, da das Gewebe zunehmender Degeneration unterworfen ist. Komplikationen wie Graft Migration, Dislokation, Endoleaks mit wiederholten Reinterventionen können die Folge dieser bis dato ungelösten Problematik sein.

US 2005/154451 A1 offenbart die Verwendung eines Stents oder Ballons, der einen Farnesyltransferaseinhibitor aufweist, zur Behandlung von Arteriosklerose.

WO 2008/067288 A2 offenbart die Verwendung eines mit einem Farnesyltransferaseinhibitor versehenen Stents oder Grafts zur Inhibierung der Reaktion auf Fremdkörper.

Brian C. Capell, "A farnesyltransferase inhibitor prevents both the onset and late progression of cardiovascular disease in a progeria mouse model", Proc. Natl. Acad. Sci. USA (2008) 105 (41): 15902-15907 offenbart die Verwendung eines Farnesyltransferaseinhibitors zur Behandlung des Hutchinson-Gilford Progeriasyndroms.

Es besteht somit das Bedürfnis, ein Produkt zu entwickeln, mit dem sich ein bereits aufgetretenes Aneurysma behandeln lässt und zudem der Ausbildung oder dein Wiederauftreten eines Aneurysmas vorgebeugt werden kann. Dies hat insbesondere bei Patienten mit einer konsekutiven Dilatation des Aneurysmahalses im Verlauf besondere Bedeutung.

### Darstellung der Erfindung

Eine Aufgabe der vorliegenden Erfindung besteht darin, ein Produkt vorzuschlagen, das eine Behandlung von kardiovaskulären Erkrankungen ermöglicht, bei der jedoch schädliche Nebenwirkungen reduziert sind. Hauptsächlich soll eine Behandlung und Prävention von Aneurysmen ermöglicht werden. Erneutes Auftreten von aneurysmatischen Veränderungen sollen nach dieser spezifischen Behandlung reduziert werden.

Erfindungsgemäß wird ein medizinisches Produkt vorgeschlagen, das in ein Blutgefäß einführbar ist und das ein Medikament für die Behandlung eines Aneurysmas aufweist. Das Medikament ist ein Farnesyltransferaseinhibitor.

In einem ersten Aspekt umfasst die vorliegende Erfindung ein medizinisches Produkt (1, 11) einführbar in ein Blutgefäß (2, 12), das ein Medikament (3, 13) zur Behandlung oder Prävention eines Aneurysmas aufweist, dadurch gekennzeichnet, dass das Medikament (3, 13) ein Farnesyltransferaseinhibitor ist, zur Verwendung bei der Behandlung oder Prävention eines Aneurysmas.

So wie im Zusammenhang der Erfindung verwendet, sollte der Begriff "Aneurysma" im weitesten Sinne als eine permanente Erweiterung des Querschnitts von Blutgefäßen verstanden werden. Bevorzugt ist bei einem Aneurysma lediglich ein lokal begrenzter Teil des Blutgefäßes erweitert. Es kann jedoch auch multipel an verschiedenen Körperstellen auftreten, was als *Morbus aneurysmaticus* bezeichnet wird. Die Erweiterungen können bei einem Aneurysma jede Form aufweisen und beispielsweise spindel- oder sackförmig ausgeprägt sein. Ein Aneurysma kann angeboren oder erworben sein.

In einer bevorzugten Ausführungsform ist das Aneurysma ein *Aneurysma verum,* ein *Aneurysma spirum,* ein *Aneurysma dissecans* oder eine Mischform daraus, insbesondere ein *Aneurysma verum.*

Ein *Aneurysma verum* ist typischerweise dadurch gekennzeichnet, dass es innerhalb der Gefäßwand entsteht, so dass die gesamte Gefäßwand mit allen Schichten (*Intima, Media* und *Adventitia*) erweitert ist. Häufig wird ein *Aneurysma verum* durch eine oder mehrere degenerative Gefäßerkrankung(en) (z.B. Arteriosklerose, Marfan-Syndrom, Ehlers-Danlos-Syndrom) und/oder ein oder mehrere periphere arterieller Verschlusskrankheit(en) hervorgerufen. Bekannte Risikofaktoren zur Bildung von Aneurysmata sind beispielsweise Hypertonie, Rauchen, Kupfermangel, Diabetes, Alkoholismus, Hypercholisterinämie und/oder hohes Alter.

Ein *Aneurysma spirum,* welches auch als *Aneurysma falsum* bezeichnet werden kann, ist typischerweise dadurch gekennzeichnet, dass es außerhalb der Gefäßwand entsteht, daher die Gefäßwandschichten *Intima* und *Media* verletzt werden, die *Adventitia* aber weitestgehend intakt bleibt. Diese Verletzung kann typischerweise durch eine stumpfe oder scharfe Verletzung hervorgerufen werden. Durch ein *Aneurysma spirum* kann eine Gefäßleckage und/oder ein pulsierendes Hämatom entstehen. Das pulsierende Hämatom kann lediglich von verdrängtem Gewebe oder aber durch eine Bindegewebskapsel umgeben sein.

Ein *Aneurysma dissecans* basiert typischerweise auf einer Dissektion, wobei sich das Blut zwischen die Schichten der Gefäßwand eindringt und diese voneinander teilt. Dadurch kann eine zweite Blutbahn (Pseudolumen) geschaffen werden. Insbesondere bei dem Auftreten eines *Aneurysma dissecans* kann es durch Kontraktion der Gefäßwandmuskulatur zu einer Ischämie des ursprünglichen Gefäßes kommen.

Ein Aneurysma im Sinne der Erfindung kann an jedem Blutgefäß auftreten, bevorzugt an Blutgefäßen, in denen ein höherer Innendruck herrscht, somit an einer Arterie oder einer Arteriole, stärker bevorzugt an einer Arterie. Beispielsweise kann ein Aneurysma ein Aneurysma an der Aorta (Aortenaneurysma), an der *Arteria carotis,* an einer Arteria einer oberen oder unteren Extremität, der *Arteria subclavia,* einer Nierenarterie, einer Hirnarterie (cerebrales Aneurysma), der *Arteria iliaca* oder einem Herzkranzgefäß sein. Bei einem Aortenaneurysma kann zwischen einem Bauchaortenaneurysma (BAA), einem abdominalen Aortenaneurysma (AAA) und einem thorakalen Aortenaneurysma (TAA) unterschieden werden. Ein Aortenaneurysma kann unter anderem ein Aneurysma der *Aorta thoracica, Aorta abdominalis,* oder ein thorakoabdominales Aortenaneurysma sein.

In einer bevorzugten Ausführungsform ist das Aneurysma ein Aneurysma an der Aorta, an der, *Arteria carotis,* an einer Arteria einer oberen oder unteren Extremität, der *Arteria subclavia,* einer Nierenarterie, einer Hirnarterie, an der *Arteria iliaca* oder an einem Herzkranzgefäß.

Zahlreiche Ursachen für die Entstehung eines Aneurysma sind dem Fachmann wohlbekannt. So kann ein Aneurysma durch eine degenerative Gefäßerkrankung (z.B. Arteriosklerose), Traumata, Infektionen (z-B. rheumatisches Fieber, Syphilis, Lyme-Borreliose), Entzündungen, angeborene Bindegewebsschwächen (z.B. Marfan-Syndrom, Ehlers-Danlos-Syndrom), eine Schwäche der Endothelzellen, Herzfehler (z.B. Rechts-Links-Shunt), das Auftreten des Kawasaki-Syndroms, durch äußere Verletzungen und/oder durch innere Verletzungen hervorgerufen werden. Bevorzugt ist das Aneurysma im Sinne der vorliegenden Erfindung ein Aneurysma einer Arterie, das durch eine arterielle Wanddegeneration hervorgerufen wird.

In einer bevorzugten Ausführungsform wird das Aneurysma durch eine Degeneration einer Gefäßwand hervorgerufen, insbesondere wobei die Gefäßwand die Wand der Aorta ist.

Eine Degeneration der Aorta, insbesondere im Bereich des Aneurysmahalses, kann durch ein vermehrtes Aufkommen von Proteasen und einen zunehmender Untergang von glatten Muskelzellen in der Muskularisschicht, die hier zur Degeneration des entsprechenden Segmentes mit konsekutiver Dilatation führen, hervorgerufen werden. Hierbei kann auch apoptotischer Zelltod in dem entsprechenden Gewebe auftreten. Die Dilatation kann altersbedingt sein und/oder durch eine Krankheit (z.B. Arteriosklerose, Marfan-Syndrom, Ehlers-Danlos-Syndrom) hervorgerufen sein.

Ein Aneurysma kann stabil oder labil sein. Ein Aneurysma kann optional von einer Bindegewebskapsel umgeben sein. Ein Aneurysma kann spontan oder infolge äußerer Einwirkung reißen (Ruptur). Eine Ruptur kann beispielsweise zu einem intrapernetonealen Hämatom (Hämaskos) und/oder zu einer Hirnblutung (z.B. Subarachnoidalblutung) führen, was zu einem Verbluten des Patienten führen kann. Ein im Gehirn lokalisiertes Aneurysma kann optional Druck auf einen Nerv ausüben und zu Lähmungserscheinungen führen. Somit kann auch ein nichtreißendes, somit intaktes, Aneurysma zum Tod durch Atemstillstand führen. Ferner kann es zu einem thrombolytischen oder embolischen Verschluss eines oder mehrerer Gefäß(e) kommen. So kann beispielsweise ein Schlaganfäll, ein Herzinfarkt oder eine Embolie in einem anderen Organ (z.B. eine Lungen- oder Nierenembolie) auftreten. Die Symptomatik kann akut oder chronisch sein. Dem Fachmann ist bekannt, dass sich die Rückstellkräfte in einem Aneurysma typischerweise umgekehrt proportional zu dessen Durchmesser verhalten, so dass ein großes Aneurysma typischerweise labiler ist. Bevorzugt ist ein Aneurysma im Sinne der Erfindung ein größeres Aneurysma, das endovaskulär zugänglich ist, somit ein

Aneurysma, das einen maximalen Durchmesser von größer als 0,2 cm, größer als 0,3 cm, größer als 0,4 cm, größer als 0,6 cm, größer als 0,8 cm, größer als 1 cm, größer als 2 cm, größer als 3 cm, größer als 4 cm, größer als 5 cm, größer als 6 cm, größer als 7 cm, größer als 8 cm, größer als 9 cm oder größer als 10 cm aufweist.

Ein Aneurysma im Sinne der Erfindung kann symptomatisch (z.B. Rückenschmerzen, Abdommalschmerzen, Nierenkolik oder Pankreatitis, Lähmnrigen, Halbseitenlähmung, Sprachausfälle, andere Hirnschäden) oder asymptomatisch sein. Auch oligosymptomatische Reaktionen sind möglich.

Die Diagnose kann durch jedes dem Fachmann bekannte Verfahren erfolgen. So kann die Diagnose beispielsweise durch Betrachtung (Inspektion), Ertastung (Palpation), Ultraschall-Untersuchung(en) (Sönographie(n), Röntgendiagnostik, Computertomographie (CT), Angiographie oder Magnetresonanztomographie (MRT) erfolgen.

Offenbart ist, dass das medizinische Produkt der vorliegenden Erfindung nicht allein zur Behandlung oder Prävention eines Aneurysmas dienen, sondern zudem zur Behandlung oder Prävention von Arterioskleroseerkrankungen verwendet werden kann. Die Behandlung oder Prävention von Arterioskleroseerkrankungen kann wiederum die Ausbildung eines Aneurysmas verhindern.

In einer bevorzugten Ausführungsform ist das medizinische Produkt (11) zur erfindutigsgemäßen Verwendung ein Angioplastie-Ballon, womit die erkrankte Stelle in dem Blutgefäß (12) mittels des Medikaments (13), Farnesyltransferaseinbibitor, behandelbar ist.

Gemäß einer weiteren Ausführungsform ist das medizinische Produkt (1) zur erfindungsgemäßen Verwendung im Blutgefäß (2) implantierbar, wobei mittels des medizinischen Produktes (1) das Medikament (3), Farnesyltransferaseinhibitor, am Blutgefäß (2) applizierbar ist.

Gemäß einer noch stärker bevorzugten Ausführungsform umfasst das medizinische Produkt (1) zur erfindungsgemäßen Verwendung einen Stent, einen Endograft oder eine Endoprothese.

Somit kann ein Gefäßimplantat etwa ein Stent, eine Endoprothese, ein Endograft, oder eine mit FTI beschichtete Prothese sein. Stents sind im Prinzip mit einem kleinen Röhrchen vergleichbar, das aus einem Drahtgeflecht besteht.

Gemäß einer anderen noch stärker bevorzugten Ausführungsform umfasst das medizinische Produkt (1) zur erfindungsgemäßen Verwendung einen Barestent und/oder einen Stentgraft.

Ein Barestent umfasst typischerweise ein rigides Drahtnetzt und/oder Drahtgeflecht, dass in der Lage ist, mechanisch ein Blutgefäß offen zu halten. Zudem kann ein Barestent innerhalb oder außerhalb seines Lumens eine Kunststoffummantelung aufweisen die durchlässig für Flüssigkeiten oder undurchlässig ist. Eine Endoprothese ist ein Drahtgeflechtimplantat mit einer integrierten Kunststoffummantelung. Bei bestimmten Varianten dieser Ausführungsformen umfasst das medizinische Produkt einen Stent, einen Endograft, eine Endoprothese, oder eine Gefäßprothese. Ein Stentgraft besteht üblicherweise aus einem Gewebe, das sich zum größten Teil aus Metall zusammensetzt. Dem Fachmann wird jedoch bekannt sein, dass ein Stentgraft auch beschichtet sein kann und/oder auch Nichtmetallkomponenten umfassen kann oder alternativ sogar aus nichtmetallenen Komponenten bestehen kann. Typischerweise ist das Gewebe eines Stentgrafts ein rigides Gewebe oder Geflecht, das in der Lage ist, mechanisch ein Blutgefäß offen zu halten.

Gemäß einer weiteren bevorzugten Ausführungsform kann das medizinisches Produkt der vorliegenden Erfindung zusätzlich zum Medikament (3, 13) einen anti-proliferativen Wirkstoff umfassen.

Gemäß einer weiteren bevorzugten Ausführungsform kann das medizinisches Produkt zur Verwendung gemäß der vorliegenden Erfindung zusätzlich zum Medikament (3, 13) einen retardierenden Hilfsstoff umfassen.

Gemäß einer weiteren bevorzugten Ausführungsform kann das medizinisches Produkt zur Verwendung gemäß der vorliegenden Erfindung zum Nachfüllen bzw. Nachbeschichten mit dem Medikament (3, 13) vorgesehen sein.

Das medizinische Produkt kann einen Ballon oder vergleichbares Produkt zur Gefäßaufweitung umfassen. Der Ballon kann ein Ende eines Katheters bilden, etwa eines Gefäßkatheters. Der Ballon kann zur Durchführung einer Ballondilatation vorgesehen sein, etwa im Rahmen einer perkutanen transluminalen Angioplastie (PTA).

Der Ballon kann Teil eines Medikamenten-beschichteten Ballonkatheters ("Drug Eluting Ballon") sein. Die Oberfläche des Ballons kann ganz oder teilweise mit dem Medikament beschichtet sein. Die Beschichtung kann ein Trägermaterial und/oder weitere Wirkstoffe wie etwa einen (weiteren) anti-proliferativen oder anti-inflammatorischen Wirkstoff umfassen.

Das medizinische Produkt kann ein Implantat umfassen. Implantate sind künstliche Produkte, die eine biologische Struktur in ihrer Funktion unterstützen oder ersetzen. Zusätzlich oder alternativ können Implantate eine Funktion als Speicher bzw. Depot für Substanzen wie etwa Medikamenten haben, so dass die Substanz nach dem Einsetzen oder Einbringen des Implantats an umliegendes biologisches Gewebe abgegeben wird.

Implantate werden häufig aus einem Metall oder einem Kunststoff (Polymer) hergestellt. Die Grundstruktur (bspw. ein Grundgerüst) des Implantats kann ganz oder zum Teil aus einem bio-inerten Material bestehen, bspw. aus einem Metall wie Titan. Das Implantat kann neben dieser Grundstruktur eine weitere Komponente umfassen. Bestimmte Varianten von Implantaten umfassen z.B. kunststoffbeschichtete Metallstrukturen.

Das für das Implantat verwendete Metall kann bspw. rostfreien Stahl, Kobalt, Chrom, das aus Nickel und Titan bestehende "Shape-Memory" Material Nitinol, Titan, oder diese Materialien umfassende Legierungen aufweisen, bspw. Titan- oder Kobalt/Chrom-Legierungen.

Für das Implantat verwendetes Material kann auch keramische Materialien oder polymerische Materialien wie Polyethylen, Polyurethan, Polyester wie z.B. Polylactide oder Silikon umfassen.

Bei dem Implantat kann es sich um ein Gefäßimplantat handeln, etwa um einen Stent. Ein Stent gleicht in vielen Fällen einem kleinen Röhrchen, welches in ein Gefäß oder einen vergleichbaren Durchgang eingebracht wird, um dieses bzw. diesen offenzuhalten, also etwa um einen Gefäßverschluss zu verhindern.

Der Durchmesser eines Stents ist meist vor der Benutzung minimiert, bspw. durch Zusammenlegen oder -klappen. Nach dem Verbringen an die gewünschte Position wird der Durchmesser des Stents vergrößert, etwa mittels eines aufblasbaren Ballons. Nach der Vergrößerung drückt sich der Stent in das umliegende Gewebe, z.B. eine Gefäßwandung, und verbleibt so an Ort und Stelle. Stents können etwa mittels Endoskopie eingebracht werden.

Stents können z.B. ein Gittergerüst aus einem Material wie Metall (Drahtgeflecht) oder Kunststoff umfassen. Das Gerüst kann beschichtet sein.

Bei dem Implantat kann es sich um eine Gefäßprothese handeln, bspw. um ein Endögraft oder eine Endoprothese handeln. Der Endograft (Stentgraft) ist eine innerhalb eines Gefäßes zu platzierende Prothese, eine Endoprothese ersetzt einen Teil eines Gefäßes. Die Gefäßprothese kann eine Grundstruktur aus einem Kunststoff wie Polyethylenterephthalat (PET) oder Polytetrafluorethylen (PTFE) umfassen, oder kann ein Metallgeflecht umfassen. Bestimmte Ausführungsformen einer Gefäßprothese umfassen ein Drahtgeflecht mit einer Kunststoffummantelung, in bzw. auf die das Medikament sowie ggf. weitere Wirkstoffe und/oder retardierende Hilfsstoffe wie Wachs oder Harz aufgenommen ist bzw. sind.

Das Medizinprodukt kann mehrere Schichten umfassen, bspw. kann ein Implantat ein metallisches Grundgerüst mit einer Beschichtung aus mindestens einem Kunststoff bzw. Polymermaterial wie etwa einem oder mehreren der vorgenannten Materialien umfassen. Unter einer Beschichtung wird allgemein verstanden, dass das Implantat auf einer Grundstruktur (einem Grundgerüst, bspw. einem Metallgeflecht) eine Schicht einer bestimmten Dicke aus einem bestimmten Material umfasst. Das Material kann ein Trägermaterial wie etwa ein Polymer, das Medikament, einen weiteren Wirkstoff oder mehrere weitere Wirkstoffe, und/oder ein retardierendes Material umfassen, welches die Abgabe des Medikaments und/oder der weiteren Wirkstoffe verzögert. Das Trägermaterial, allgemeiner das Beschichtungsmaterial insgesamt kann porös sein und so das erfindungsgemäße Medikament (und ggf. weitere Wirkstoffe wie etwa Entzündungshemmer) aufnehmen bzw. umfassen.

Das Medizinprodukt kann beschichtet sein, um ein Medikament aufzunehmen und in geeigneter Weise verzögert wieder freizusetzen. Hierbei kann es sich um eine Beschichtung mit einem Polymer (z.B. als Trägermaterial) handeln.

Das Medizinprodukt zur Verwendung gemäß der vorliegenden Erfindung kann neben dem eigentlichen Medikament mindestens einen zusätzlichen Wirkstoff enthalten. Der weitere Wirkstoff kann vorgesehen werden, um die anti-proliferative Wirkung zu verstärken (bspw. kann der weitere Wirkstoff Paclitaxel umfassen). Ein weiterer Wirkstoff kann vorgesehen werden, um Entzündungen zu vermeiden, die etwa durch die verwendeten Beschichtungsmaterialien hervorgerufen werden könnten; ein derartiger Wirkstoff kann also etwa einen anti-inflammatorischen Wirkstoff umfassen. Neben einem weiteren anti-proliferativen Wirkstoff und/oder einem anti-inflammatorischen Wirkstoff können zusätzlich oder alternativ ein antibiotischer Wirkstoff, ein antithrombotischer Wirkstoff, etc. vorliegen.

Bei bestimmten Varianten des erfindungsgemäßen Medizinprodukts zur Verwendung gemäß der vorliegenden Erfindung kann ein- und dieselbe Beschichtung (z.B. ein Polymer umfassend) zur Speicherung des Medikaments zusammen mit den zusätzlichen Wirkstoffen versehen sein. Alternativ sind mehrere Beschichtungen aus unterschiedlichen Polymeren denkbar, die nebeneinander oder übereinander vorliegen und wobei eine Beschichtung nur mit dem Medikament und eine andere Beschichtung nur mit dem weiteren Wirkstoff versehen ist.

Um Kunststoffbeschichtungen zu vermeiden, können auch andere ah sich bekannte Ansätze zur Bereitstellung mikroporöser Stentoberflächen zur Aufnahme und verzögerten Wiederabgabe eines Medikaments verwendet werden.

Bestimme Ausführungsformen des erfindungsgemäßen Medizinprodukts zur Verwendung bei der Behandlung oder Prävention eines Aneurysmas sind mit einer zusätzlichen, d.h. weiteren oder zweiten Beschichtung versehen, um die retardierte Abgabe des Medikaments in geeigneter Weise zu beeinflussen, z.B. zu verzögern. Diese zusätzliche Beschichtung kann bspw. ein Beschichtungsmaterial wie Wachs oder Harz umfassen, das vorzugsweise biokompatibel ist. Die zusätzliche Beschichtung kann als zusätzliche, bspw. äußerste Schicht vorliegen oder kann zusammen mit einem Trägermaterial, dem Medikament und ggf. weiteren Wirkstoffen in gemeinsamer Raumerfüllung vorliegen.

Offenbart wird weiterhin ein Verfahren zum Vorbereiten eines Medizinproduktes, bei dem das Medizinprodukt mit einem Medikament versehen wird. Das Medikament ist ein Farnesyltransferaseinhibitor wie hier beschrieben.

Das Verfahren kann den vorgelagerten Schritt enthalten, bei dem ein Ballon oder eine Grundstruktur eines Implantats mit einem Beschichtungsmaterial beschichtet wird. Das Beschichtungsmaterial ist zur Aufnahme des Medikaments vorgesehen und bspw. in geeigneter Weise mikroporös.

Bestimmte Ausführungsformen des Verfahrens enthalten den nachgelagerten Schritt des Aufbringens einer zusätzlichen Beschichtung, um die Abgabe des Medikaments zu verzögern.

Das Medikament kann bspw. in Lösung vorliegen und kann mittels Sprühen bzw. einer Spraytechnologie auf das Medizinprodukt aufgebracht werden. Bei bestimmten Ausführungsformen des Verfahrens liegen ein oder mehrere Beschichtungsmaterialien und das Medikament und/oder weitere Wirkstoffe gemeinsam in einem Lösungsmittel gelöst vor und werden gemeinsam auf eine Grundstruktur wie einen Ballon oder ein Implantat-Grundgerüst aufgesprüht.

Das Aufbringen durch Sprühen kann mehrere Sprühvorgänge umfassen, bspw. einen ersten (Basis-)sprühvorgang und einen zweiten (Abschluss-)sprühvorgang. Hierbei kann die versprühte Substanz die gleiche Zusammensetzung oder unterschiedliche Zusammensetzung haben; bspw. kann in einem ersten Sprühvorgang hauptsächlich oder nur das Medikament, ggf. mindestens ein weiterer Wirkstoff versprüht werden, und in einem nachfolgenden Sprühvorgang kann hauptsächlich oder nur ein retardierender Hilfsstoff versprüht werden.

Nach dem Einsprühen kann das Verfahren einen Trocknungsschritt umfassen, der eine passive oder aktive Trocknung bspw. durch Luft oder eine andere Gaszusammensetzung, und/oder eine Erwärmung des Medizinprodukts bspw. durch Erwärmung der Umgebung und/oder Applikation von Strahlung wie Infrarotstrahlung umfassen kann. Werden mehrere Sprühvorgängen durchgeführt, können ggf. dazwischen ein oder mehrere Trocknungsschritte vorgesehen werden.

In einem weiteren Aspekt umfasst die Erfindung einen Farnesyltransferaseinhibitor zur Verwendung in einem Verfahren zur Behandlung oder Prävention eines Aneurysmas.

Gemäß einer bevorzugten Ausführungsform ist dabei das Aneurysma wie oben definiert.

Die Behandlung mit einem Farnesyltransferaseinhibitor kann vor, während oder nach der chirurgischen oder endovaskulären Behandlung des Aneurysmas erfolgen. Vorzugsweise erfolgt die Behandlung mit einem Farnesyltransferaseinhibitor während oder nach der chirurgischen oder endovaskulären Behandlung des Aneurysmas.

Der Farnesyltransferaseinhibitor kann lokal oder systemisch verabreicht werden. Er kann beispielsweise injiziert werden, oral, subkutan, perkutan oder nasal verabreicht werden oder in einem medizinischen Produkt im Sinne der Erfindung vorhanden sein.

In einer bevorzugten Ausführungsform ist der Farnesyltransferaseinhibitor in einem medizinischen Produkt (1, 11) der vorliegenden Erfindung enthalten.

Dieses medizinische Produkt kann direkt zur Behandlung eines Aneurysmas verwendet werden. Alternativ oder zusätzlich kann zunächst die chirurgischen oder endovaskulären Behandlung des Aneurysmas erfolgen und nachträglich ein zweites medizinische Produkt, das den Farnesyltransferaseinhibitor enthält, zur Nachbehandlung eingesetzt werden. Ein zur Nachbehandlung eingesetztes medizinisches Produkt ist vorzugsweise ein Ballon.

In einer alternativen bevorzugten Ausführungsform wird der Farnesyltransferaseinhibitor zur Nachbehandlung eines Aneurysmas verwendet.

Wie hierin verwendet, sollte der Begriff "Nachbehandlung" im weitesten Sinne als eine Behandlung nach der Behebung einer akuten Gefahr verstanden werden. In de Regel ist die Nachbehandlung präventiv, um ein weiteres Auftreten eines Aneurysmas zu verhindern. Die Nachbehandlung kann jedoch auch dem schnelleren Abheilen dienen und/oder einer Nebenwirkungen der Behandlung eines Aneurysmas verhindern oder minimierten. Somit kann beispielsweise eine Infektionsgefahr minimiert werden.

Die Nachbehandlung kann mitmedizinischen Produkt zur erfindungsgemäßen Verwendung erfolgen.

Alternativ kann jedoch im Rahmen der erfindungsgemäßen Nachbehandlung der Farnesyltransferaseinhibitor unabhängig von dem erfindungsgemäßen medizinischen Produkt verabreicht werden. Besonders bevorzugt ist hier eine lokale Verabreichung, insbesondere mit einem Ballonkatheter. Alternativ kann der Farnesyltransferaseinhibitor auch intravenös verabreicht werden.

Wie hierin verwendet, sollte unter dem Begriff "Farnesyltransferaseinhibitor" im weitesten Sinne ein Molekül verstanden werden, das die enzymatisch katalysierte Übertragung eines Farnesylrests auf ein Substrat verhindert. Hierbei ist das Substrat, das farnesyliert wird, typischerweise ein Polypeptid von wenigstens vier Aminosäuren Länge. Ein Polypeptid umfasst vorzugsweise einen linearen Strang von Aminosäuren, welche übers Amidbindungen miteinander verknüpft sind. Ein Polypeptid kann wenigstens vier, mehr als vier, mehr als zehn, mehr als 20, mehr als 30, mehr als 50, mehr als 100, mehr als 200, mehr als 300, mehr als 400, mehr als 500, oder sogar mehr als 600 Aminosäuren lang sein. Typischerweise wird ein Polypeptid, welches mehr als ein Sekundärstrukturelement umfasst als Proteindomäne, ein Polypeptid, welches eine komplexe Tertiärstruktur aufweist, als Protein bezeichnet. Ein Polypeptid kann jedoch auch ein verzweigtes Polypeptid sein, bei dem eine oder mehrere Aminosäure(n) an die Seitenketten anderer Aminosäuren konjugiert ist/sind. Das Polypeptid kann auch posttranslationale Modifikationen aufweisen. Zahlreiche posttranslationale Modifikationen sind dem Fachmann gut bekannt und umfassen beispielsweise Lipidierung, Phosphorylierung, Sulfatierung, Glykosylierung, Verkürzung (Trunkierung), Zyklisierung mehrerer Aminosäurereste, Zyklisierung des Polypeptidstrangs, Oxidation, Reduktion, Dekarboxylierung, Acetylierung/Azetylierung, Amidierung, Deamidierung, Ausbildung einer Disulfidbrücke, Ausbildung von Pyroglutamat, Anfügung einer oder mehrerer Aminosäure(n), Anfügung eines oder mehrerer Kofaktoren (z.B. Biotinylierung, Konjugation einer Hämgruppe), Komplexierung von Metallionen, Nichtmetallionen, Peptiden, kleinmolekularen Molekülen und/oder Konjugation von Eisen-Schwefel-Clustern. Zudem können weitere Kofaktoren gebunden sein wie beispielsweise ATP, ADP, NAD+, NADH+H+, NADP+, NADPH+H+, Metallionen, Anionen oder Lipide. Vorzugsweise umfasst ein Polypeptid, das enzymatisch katalysiert farnesyliert wird, ein CAAX-Sequenzmotiv, wobei C einen Cysteinrest repräsentiert, A einen aliphatische Aminosäurerest und X einen weiteren Aminosäurerest, der von dem die Farnesylierung katalysierenden Enzym erkannt wird.

Wie hierin verwendet, beschreibt die enzymatisch katalysierte Übertragung eines Farnesylrests eine biochemische Rektion, bei der ein Farnesylrest auf ein Substrat, vorzugsweise ein Polypeptid, übertragen wird. Ein Enzym, das die Übertragung eines Farnesylrests auf ein Substrat katalysiert, wird als Färnesyltransferase bezeichnet. Typischerweise wird hierbei aktiviertes Farnesol übertragen. Aktiviertes Farnesol ist vorzugsweise Farnesyldiphosphat (Famesylpyrophosphat, FPP).

Vorzugsweise wird das Polypeptid, das das Substrat darstellt, an einem Cysteinrest farnelysiert. Hierbei wird ein Thiolester gebildet. Die Begriffe "Thiolester" und "Thioester" sind austauschbar und beschreiben eine R₁-CO-S-R₂-Gruppe, wobei ein Thiolester auch die tautomere Form des Esters R₁-COH=S-R₂ umfassen kann. Bevorzugt befindet sich der Cysteinrest, der famelysiert werden kann nahe des C-terminalen Endes des Proteins. Besonders bevorzugt wird der Cysteinrest eines CAAX-Sequenzmotivs farnesyliert, wobei C einen Cysteinrest, A einen aliphatische Aminosäurerest und X einen weiteren Aminosäurerest, der von dem die Farnesylierung katalysierenden Enzym erkannt wird, repräsentiert.

Das Enzym, das die Farnesylierung katalysiert, ist vorzugsweise eine Farnesyltransferase (FTase), die eine Prenylttansferase der Enzymklassifikationsnummer EC 2.5.1.X, stärker bevorzugt EC 2.5.1.29, EC 2.5.1.58 oder EC 2.5.1.59, noch stärker bevorzugt EC 2.5.1.29 oder EC 2.5.1.58 darstellt. Typischerweise bindet das Enzym ein oder mehrere Zinkion(en) (Zn²⁺) Als Farnesyltransferase im Sinne der Erfindung kann auch Geranylgeranyltransferase wirken, da auch dieses Enzym in der Lage ist, bestimmte Polypeptide zu farnelysieren.

Ein Farnesyltransferaseinhibitor kann jeder Stoff oder jede molekulare Zusammensetzung sein, der/die in der Lage ist, die enzymatisch katalysierte Farnesylierung zu verlangsamen oder zu verhindern. Vorzugsweise ist unter einer Verlangsamung der Farnesylierungsgeschwindigkeit eine Verlangsamung um mehr als 10%, stärker bevorzugt um mehr als 25%, noch stärker bevorzugt um mehr als 50%, noch stärker bevorzugt um mehr als 75%, noch stärker bevorzugt um mehr als 80%, noch stärker bevorzugt um mehr als 90%, und am stärksten bevorzugt um mehr als 95% durch den Zusatz des Farnesyltransferaseinhibitors in einer geeigneten Konzentration am Wirkort im Vergleich zu einer gleichartigen Reaktionsumgebung ohne den Zusatz des Farnesyltransferaseinhibitors zu verstehen. Eine geeignete Konzentration des Farnesyltransferaseinhibitors am Wirkort kann vorzugsweise bei unter 10 mM, unter 1 mM, unter 100 µM, unter 10 µM, unter 1 µM, unter 100 nM, unter 10 nM oder unter 1 nM liegen. Unter der Konzentration am Wirkort ist die Konzentration an Farnesyltransferaseinhibitor in der unmittelbaren Umgebung des zu behandelnden Gewebes zu verstehen. Die Konzentration am Wirkort kann sich von der systemischen Konzentration des Farnesyltransferaseinhibitors, die üblicherweise im Blut gemessen wird, erheblich unterscheiden.

Der Farnesyltransferaseinhibitor kann ein Antimetabolit sein, so wie beispielsweise ein Analogon des Farnesols, des Farnesylphosphats, des Farnesyldiphosphats oder eines Substratpeptids. Der Farnesyltransferaseinhibitor kann auch ein Molekül mit andersartiger Struktur sein, das in die Bindungstasche des Peptidsubstrats oder des Farnesyldipihosphats binden kann. Alternativ kann der Farnesyltransferaseinhibitor ein allosterischer Inhibitor sein.

Der Farnesyltransferaseinhibitor kann eine beliebige molekulare Struktur aufweisen. Er kann beispielsweise ein peptidischer Wirkstoff, ein Peptidomimetikum oder ein nichtpeptidomimetischer kleinmolekularer Wirkstoff sein. Ein peptidischer Wirkstoff besteht größtenteils aus einem Peptid. Das Peptid kann jedoch an andere molekulare Strukturen konjugiert sein, wie beispielsweise ein organisches, biologisch verträgliches Polymer (z.B. Polyethylenglykol (PEG), Polyethylenimin (PEI), Hydroxypropylmethylacrylamid (HPMA), ein Lipid, einen Alkylrest oder ein anderes Polypeptid. Ein Peptidomimetikum ist ein Wirkstoff dessen molekulare Struktur einem Peptid nachempfunden ist. Ein Peptidomimetikum kann beispielsweise beta-Amininosäuren (β-Amininosäuren), gamma-Amininosäuren (γ-Amininosäuren) oder D-Aminosäuren enthalten oder aus diesen oder einer Kombination aus mehreren dieser bestehen. Auch ein Peptidomimetikum kann an andere molekulare Strukturen konjugiert sein, wie beispielsweise ein organisches, biologisch verträgliches Polymer konjugiert sein. Ein Peptidomimetikum kann auch ein retroinverses Peptid sein. Ein kleinmolekularer Wirkstoff ist ein Molekül mit einem Molekulargewicht von weniger als 1500 Da, vorzugsweise weniger als 1000 Da, noch stärker bevorzugt weniger als 500 Da. Auch ein kleinmolekularer Wirkstoff kann an andere molekulare Strukturen konjugiert sein, wie beispielsweise ein organisches, biologisch verträgliches Polymer konjugiert sein.

Der Farnesyltransferaseinhibitor kann beispielsweise R11577 (Zarnestra, Tipifarnib), SCH66336 (Lonafarnib), FTI-277, GGTI-298, BMS-214664, L-778, oder L-123 sein. Besonders bevorzugt ist der Wirkstoff R11577. R11577 ist ein Imidazol enthaltener heterozyklischer, nichtpeptidomimetischer Wirkstoff. R11577 ist auch als Zarnestra bekannt und stellt ein zugelassenes Medikament dar. R11577, kann beispielsweise in Tierexperimenten, in denen eine orale Verabreichung praktiziert wurde, eine Verhinderung der Progression der kardiovaskulären Erkrankung bewirken (Capell BC, Olive M, Erdos MR, Cao K, Faddah DA, Tavarez UL, Conneely KN, Qu X, San H, Ganesh SK, Chen X, Avallone H, Kolodgie FD, Virmani R, Nabel EG, Collins FS., Proc Natl Acad Sci U S A, Oktober 2008). Die orale Applikation von Orale Applikation von Medikamenten in den tierexperimentellen Untersuchungen konnte bereits mit systemischern Einsatz von Farnesyltransferaseinhibitor, ZARNESTRA, die Progression der kardiovaskulären (arteriosklerotische) Erkrankung verhindert werden. Diese Anwendung ist hilfreich für die Behandlung von kardiovaskulären Erkrankungen, sie hat jedoch schädliche Nebenwirkungen, die nicht vertretbar sind.

Farnesyltransferaseinhibitoren sind bereits als zugelassene Therapeutika (ZARNESTRA) in der onkologischen Therapie eingesetzt. Sie zeigen eine schwache Toxizität und es wird bei oraler Applikation gut vertragen.

Der Farnesyltransferaseinhibitor kann an die Oberfläche des medizinischen Produkts kovalent oder nichtkovalent gebunden sein oder an die Oberfläche angelagert sein. Ferner kann der Farnesyltransferaseinhibitor in das medizinische Produkt eingebettet sein. Eine kovalente Bindung kann dadurch erfolgen, dass der Farnesyltransferaseinhibitor direkt an die Oberfläche des medizinischen Produkts gebunden ist oder aber über einen molekularen Abstandhalter (Linker/Spacer) gebunden ist. Ein Linker kann jedes biologisch verträgliche Material sein, wie beispielsweise Polyethylenglykol (PEG), ein peptidischer Linker, ein Protein, ein Alkyllinker ein HPMA-Linker oder ein PLGA-Linker. Der Linker kann biologisch inert oder biologisch abbaubar sein. Der Linker kann kovalent oder nicht kovalent mit dem Farnesyltransferaseinhibitor und/oder dem medizinischen Produkt verknüpft sein, beispielsweise über eine Veresterung, Maleimidkopplung, oder Amidbindung. Eine nichtkovalente Binding kann die Bindung des Farnesyltransferaseinhibitors selbst oder eines mit dem Farnesyltransferaseinhibitor verbundenen Linkers mit der Oberfläche des medizinischen Produkts sein, die durch ionische Wechselwirkung, Wasserstoffbrücken oder Komplexbindung hervorgerufen wird. Beispiele für eine nichtkovalente Bindung können die Wechselwirkung zwischen einer Streptavidin-beschichteten Oberfläche und einem mit Biotin konjugierten Farnesyltransferaseinhibitor oder die Wechselwirkung zwischen einem mit einem Histidinschwanz (His-Tag) verbundenen Farnesyltransferaseinhibitor und einer Nickel-NTA-konjugierten Oberfläche. Ferner kann auch ein GST-Tag oder ähnliches verwendet werden. Der Farnesyltransferaseinhibitor kann auch über Wechselwirkungen, wie beispielsweise hydrophobe Wechselwirkungen an die Oberfläche angelagert werden. Ferner kann der Farnesyltransferaseinhibitor in das medizinische Produkt eingebettet werden, insbesondere dann, wenn dieses porös oder semiporöse Eigenschaften aufweist, somit Poren aufweist, in die der Farnesyltransferaseinhibitor aufgenommen werden kann, oder der Farnesyltransferaseinhibitor während der Herstellung des medizinischen Produkts in letzteres eingegossen wird. Das medizinische Produkt kann auch aus einem biologisch abbaubaren Material bestehen, dass sich im Körper des Patienten zersetzt und dann den Farnesyltransferaseinhibitor freisetzt oder mit einem biologisch abbaubaren Material beschichtet sein, dass sich im Körper des Patienten zersetzt und dann den Farnesyltransferaseinhibitor freisetzt. Ein solches biologisch abbaubares Material ist beispielsweise Polymilchsäure (polylactic acid (PA)), Polyglykolsäure (glycolic acid (GA)), Polymilchsäure-Glykolsäure-Kopolymere (poly(lactic-co-glycolic acid) (PLGA)), Polycaprolacton, Polyglycolid, Poly-3-Hydroxybutyrat oder Kopolymere aus zwei oder mehrerer dieser. Der Farnesyltransferaseinhibitor kann auch als Mikropartikel und/oder Nanopartikel gebunden sein, die mit der medizinischen Material assoziiert sind.

Der Farnesyltransferaseinhibitor kann in jeder geeigneten pharmazeutischen Zusammensetzung vorliegen. Die pharmazeutische Zusammensetzung kann ein oder mehrere Hilfsstoffe und/oder Trägerstoffe enthalten, wie beispielsweise ein oder mehrere Lösungsmittel (z.B. Wasser, Dimethylsulfoxid (DMSO), Ethanol, pflanzliches Öl, tierisches Öl, Mineralöl, Parafinöl), ein oder mehrere oberflächenaktive Sustanz(en) (z.B. Natriumlaurylsulfat (sodium dodecyl sulfate (SDS)), ein oder mehrere Farbstoff(e) (z.B. TiO₂, Nahrungsmittelfarben, Fluoreszein, Fluoreszeonderivate, Cy-Farbstoff(e), Alexa Fluor-Farbstoff(e), S-Farbstoff(e), Rhodamin, Quantum Dots), ein oder mehrere Schaumbildner, ein oder mehrere Vitamin(e), ein oder mehrere Salz(e) (z.B. Natrium-, Kalium-, Magnesium-, Kalzium oder Zinksalze), ein oder mehrere Verdickungsmittel (z.B. Carboxymethylzellulose (CMC), Polyethylenglycol (PEG), Sorbitol), ein oder mehrere Feuchthaltemittel (z.B., Sorbitol, Glycerol, Mannitol, Propylenglycol, Polydextrose), ein oder mehrere weitere(s) Enzym(e), ein oder mehrere Konservierungsmittel (z.B. Benzoesäure, Methylparaben), ein oder mehrere Emulgator(en), ein oder mehrere Füllstoff(e), ein oder mehrere Trennmittel, ein oder mehrere antioxidantische(s) Mittel, ein oder mehrere Kräuterextrakt(e), ein oder mehrere organisch(e) bioverträgliche(s) Polymer(e) (z.B. Hydroxypropylmethacrylamid (HPMA), Polyethylenimin (PEI), Carboxymethylzellulose (CMC), Polyethylenglycol (PEG)), ein oder mehrere Aufnahmevermittler (z.B. Polyethylenimin (PEI), Dimethylsulfoxid (DMSO), zellpenetrierende(s) Peptid(e) (CPP), Proteintransduktionsdomäne(n) (PTD), antimikrobielle Peptide), ein oder mehrere Süßstoff(e) (z.B. Sucröse, Acesulfam K, Saccharin), ein oder mehrere homöopathische Zusätze, ein oder mehrere Geschmacksstoffe und/oder ein oder mehrere Duftstoff(e).

Der Farnesyltransferaseinhibitor kann lokal oder systemisch verabreicht werden. Eine systemische Verabreichung kann beispielsweise eine orale (z.B. als Pulver, Tablette, Pille, Kapsel, Zerbeißkapsel, Sirup, oder Flüssigkeit), eine intramuskuläre (z.B. über eine Spritze, Kanüle), eine intravenöse (z.B. über eine Spritze, Kanüle), eine intraarterielle (z.B. über eine Spritze, Kanüle), eine subkutane (z.B. über eine Spritze, Kanüle, ein Reservoir (z.B. eine PLGA-Reservoir)), eine perkutane (z.B. als Pflaster, Creme, Salbe) oder eine nasale Verabreichung sein. Eine lokale Verabreichung kann eine Verabreichung über das medizinische Produkt sein. Das medizinische Produkt kann den Farnesyltransferaseinhibitor enthalten, indem dieser im Material des medizinischen Produkts, optional in einer pharmazeutischen Zusammensetzung, eingebettet ist. Der Farnesyltransferaseinhibitor kann gelöst als Flüssigkeit oder Sirup, oder als Trockensubstanz vorliegen. Er kann getrocknet oder gefriergetrocknet sein.

Außerdem wird ein Farnesyltransferaseinhibitor zur Verwendung in einem Verfahren zur Behandlung oder Prävention von Arteriosklerose offenbart, wobei der Farnesyltransferaseinhibitor in einem medizinischen Produkt (1, 11) gemäß der Erfindung enthalten ist.

In einer bevorzugten Ausführungsform wird der Farnesyltransferaseinhibitor lokal verabreicht und das medizinische Produkt (1,11) wird an der erkrankten Stelle platziert und/oder fixiert und der Farnesyltransferaseinhibitor an der erkrankten Stelle abgegeben.

Wie hierin verwendet, bedeutet Platzieren vorzugsweise das Einbringen des medizinischen Produkts in ein Gefäß. Hier kann es temporär verweilen, wie dies beispielsweise bei einem Ballonkatether regelmäßig der Fall ist, oder aber fixiert werden. Das Fixieren umfasst unter Anderem das Anpressen an die Gefäßwand, das optionale Einnähen des medizinischen Produkts in ein Gewebe und/oder das Einwachsen des medizinischen Produkts in ein Gewebe.

In einer weiteren bevorzugten Ausführungsform ist der Farnesyltransferaseinhibitor R11577, SCH66336, FTI-277, GGTI-298, BMS-214664, L-778 oder L-123. Noch stärker bevorzugt ist der Farnesyltransferaseinhibitor R11577.

Der Farnesyltransferaseinhibitor kann mit anderen Therapien kombiniert werden, wie beispielsweise Ultraschallbehandlung, Bestrahlung (z.B. Ultraviolettbestrahlung (UV-Bestrahlung), Röntgenbestrahlung, Röntgenweichbestrahlung, radioaktive Bestrahlung (z.B. α-Strahlung, β-Strahlung, γ-Strahlung), kosmische Strahlung), Behandlung mit einem oder mehreren Proliferationsinhibitoren (z.B. Alkylanz (Cisplatin, Carboplatin, Satraplatin, Oxaliplatin, Mechlorethamin, Cyclophosphamid, Chlorambucil, Ifosfamid), Signaltransduktionsinhibitor, Vincaalkaloid (z.B. Vincristin, Vinblastin, Vinorelbin, Vindesin), Taxan, Podophyllotoxin, Topoisomeraseinhibitor, Antimetabolit (z.B., Methotrexat, Dihydrofolat, 5-Fluorouracil), Antineoplastisches Agenz). Ferner kann der Farnesyltransferaseinhibitor auch entzündungshemmende Mittel enthalten (z.B. Acetylsalicylsäure (ASS), Benzydaminhydrochlorid, Bufexamac, Diclofenac, Diflunisal, Flurbiprofen, Ibuprofen, Indometacin, Ketoprofen, Mefenaminsäure, Metamizol, Naproxen, Oxyphenbutazon, Phenylbutazon, Phenazon, Piroxicam, Propyphenazon, Salicylamid, Tarenflurbil, Tiaprofensäure, Tenoxicam, Tolfenaminsäure, Glukokortikoide, Betamethasonvalerat, Clobetasolpropionat, Dexamethason, Hydrocortison, Hydrocortisonacetat, Prednicarbat, Prednisolon, Prednison, Triamcinolonacetonid, Budesonid, Fluticason, Montelukast, Weidenrinde, Kamillenblüten, Ringelblumenblüten, Arnikablüten, Teufelskrallenwurzel, Eschenrinde, Pappelrinde, Goldrutenkraut, Rosskastanie, Weihrauch). Es wird vom Fachmann verstanden werden, dass auch weitere pharmazeutisch aktive Mittel mit dem medizinischen Produkt assoziiert sein können. Ferner wird der Fachmann verstehen, dass die vorgenannten Mittel auch direkt oder über Linker mit dem medizinischen Produkt verbunden sein können und/oder an Mikro- oder Nanobeads gebunden sein können.

Bis dato wurden Farnesyltransferaseinhibitoren lediglich zur Behandlung der Tumorbehandlung eingesetzt. Eine vaskuläre, endovaskuläre oder systemische Anwendung für die Behandlung von aneurysmatischen Erkrankungen fand bisher nicht statt.

Die obengenannte Aufgabe wird in einer Ausführungsform erfindungsgemäß dadurch gelöst, dass ein medizinisches Produkt, das ein Implantat, beispielsweise ein Stent, Stentgraft, eine Gefäßprothese oder ein Ballon ist, zur Behandlung von Aneurysmen in ein Blutgefäß eingeführt wird. Das Implantat weist ein Medikament auf, das ein Farnesyltransferaseinhibitor ist. Der Inhibitor wirkt auf die aneurysmatische Erkrankung und bessert diese.

In der Therapie eines Aneurysmas wird mit lokalen Applikation der Farnesyltransferaseinhibitoren ein völlig neues zelluläres Ziel mit Normalisierung der Zellfunktion der Gefäßwand erzielt.

Aus diesen Erkenntnissen heraus ist die lokale vasküläre bzw. endovaskuläre Applikation von Farnesyltransferaseinhibitoren in Form von damit beschichtetem Ballons, Stents, Endografts bzw. Gefäßprothesen eine wirksame therapeutische Option.

### Kurzbeschreibung der Abbildungen

Ausführungsbeispiele des medizinischen Produkts zur erfindungsgemäßen Verwendung werden anhand der folgenden Zeichnungen beschrieben. Es zeigt:
Fig. 1 eine schematische 3D Darstellung des medizinischen Produktes als ein Längsquerschnitt durch das Blutgefäß; und
Fig. 2 eine schematische 2D Darstellung des medizinischen Produktes als ein Längsquerschnitt.

### Detaillierte Beschreibung der Ausführungsbeispiele

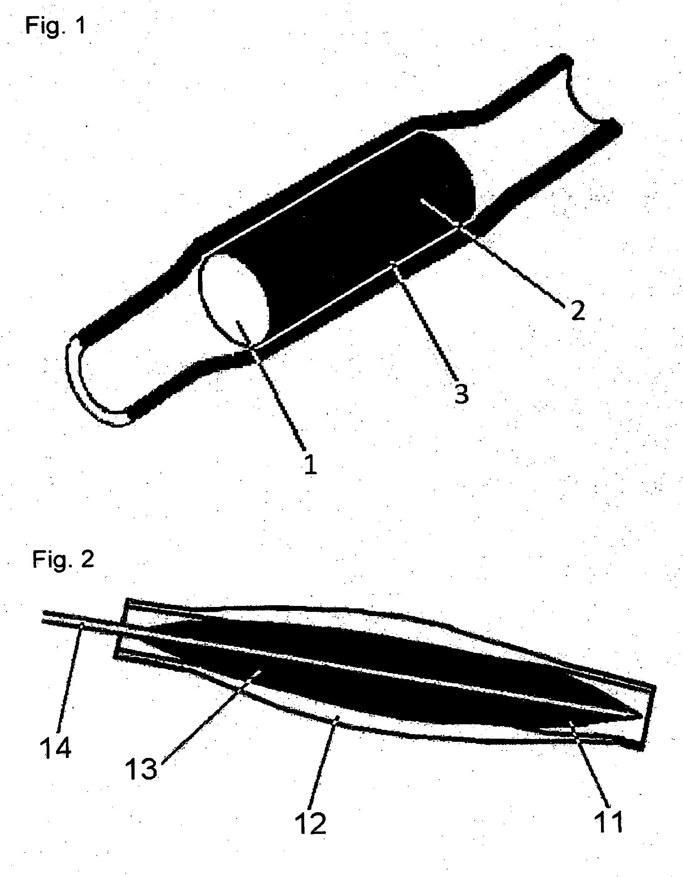

In einer beispielhaften Ausführungsform weist das medizinische Produkt 1 ein Medikament 3 auf, das ein Farnesyltransferaseinhibitor ist. Wie in Fig. 1 dargestellt, wird das Medizinische Produkt 1 in ein Blutgefäß 2 eingeführt und auf der aneurysmatisch erkrankten Stelle appliziert. Das Medikament 3 wirkt auf die aneurysmatischen Gefäßläsionen und stabilisiert sie.

Das medizinische Produkt 1 kann ein Stent, eine Gefäßprothese, ein Endograft oder ein anderes Implantat sein, das das Medikament 3 aufweist.

Das medizinische Produkt 1 kann im Medikament 3 eingetränkt oder mit dem Medikament 3 beschichtet oder damit verbunden sein.

Der Stent 1 besteht aus einem Metallgeflecht, das von einem mikroporösen Polymermaterial ummantelt ist. Bei der Herstellung des Stents 1 wurden die Hohlräume des Materials durch Sprühen oder Tränken mit einem Farnesyltransferaseinhibitor sowie Paclitaxel als weiterem anti-proliferativen Wirkstoff verfüllt. Als retardierender Hilfsstoff ist ein Wachs auf den Stent 1 aufgebracht (in Fig. 1 nicht explizit hervorgehoben).

Nach dem Einbringen in das Gefäß 2 und dem Verankern (Ausdehnen) des Stents 1 an der zu behandelnden Stelle verhindert der Stent 1 zusammen mit den enthaltenen Wirkstoffen einen (ggf. erneuten) Gefäßverschluss.

Bei anderen Ausführungsbeispielen kann, ggf. in Abhängigkeit von einem konkreten Krankheitsbild, der Farnesyltransferaseinhibitor auch ohne weiteren anti-proliferativen Wirkstoff zum Einsatz kommen.

Die Art und Menge des zugesetzten retardierenden Hilfsstoffes kann je nach konkretern Anwendungsfall gewählt werden.

In einer anderen beispielhaften Ausführungsform ist das medizinische Produkt 11 ein Ballon, der ein Ende eines Katheters 14 bildet. Der Ballon 11 weist das Medikament 13 auf und wird zu therapeutischen Anwendungen in das Blutgefäß 12 eingerührt und an der aneurysmatischen erkrankten Stelle dilatiert (Fig. 2). Die Verengung wird somit erweitert.

Der Ballon 11 ist bei der Herstellung mit dem Medikament 13 getränkt worden. Der Ballon 11 ist dadurch auf seiner gesamten Oberfläche mit dem Farnesyltransferaseinhibitor versehen. In anderen Ausführungsbeispielen ist der Ballon nur teilweise mit dem Medikament versehen, bspw. in seinem Mittelteil, nicht aber in seinen proximalen bzw. distalem Abschnitt (bspw. angrenzend bzw. gegenüber einer Kathetermündung).

Ein weiterer Wirkstoff wie bspw. Paclitaxel als anti-proliferativer Wirkstoff ist zusammen mit dem Farnesyltransferaseinhibitor auf die Oberfläche des Ballons 11 aufgebracht worden. Ein retardierender Hilfsstoff ist nicht vorgesehen, weil die therapeutisch wirksamen Substanzen während der vergleichsweise kurzen Zeit in das Gewerbe übertreten sollen, in welcher der Ballon 11 die zu behandelnde Stelle am Gefäß 12 erreicht und ausweitet.

## Patentansprüche

1. Medizinisches Produkt (1, 11) einführbar in ein Blutgefäß (2, 12), das ein Medikament (3, 13) zur Behandlung oder Prävention eines Aneurysmas aufweist, **dadurch gekennzeichnet, dass** das Medikament (3, 13) ein Farnesyltransferaseinhibitor ist, zur Verwendung bei der Behandlung oder Prävention eines Aneurysmas.

2. Medizinisches Produkt zur Verwendung gemäß Anspruch 1, wobei das Aneurysma durch eine Degeneration einer Gefäßwand hervorgerufen wird, insbesondere wobei die Gefäßwand die Wand der Aorta ist.

3. Medizinisches Produkt zur Verwendung gemäß Anspruch 1 oder 2, wobei das Aneurysma ein *Aneurysma verum,* ein *Aneurysma spirum,* ein *Aneurysma dissecans* oder eine Mischform daraus ist, insbesondere wobei das Aneurysma ein *Aneurysma verum* ist.

4. Medizinisches Produkt zur Verwendung gemäß mindestens einem der Ansprüche 1 bis 3, wobei das Aneurysma ein Aneurysma an der Aorta, an der *Arteria carotis,* an einer Arteria einer oberen oder unteren Extremität, der *Arteria subclavia,* einer Nierenarterie, einer Hirnarterie, an der *Arteria iliaca* oder an einem Herzkranzgefäß ist.

5. Medizinisches Produkt zur Verwendung gemäß mindestens eines der vorhergehenden Ansprüche, wobei das medizinische Produkt (11) ein Angioplastie-Ballon ist, womit die erkrankte Stelle in dem Blutgefäß (12) mittels des Medikaments (13), Farnesyltransferaseinhibitor, behandelbar ist.

6. Medizinisches Produkt (1) zur Verwendung gemäß mindestens eines der vorhergehenden Ansprüche, wobei das medizinische Produkt (1) im Blutgefäß (2) implantierbar ist, wobei mittels des medizinischen Produktes (1) das Medikament (3), Farnesyltransferaseinhibitor, am Blutgefäß (2) applizierbar ist.

7. Medizinisches Produkt (1) zur Verwendung gemäß Anspruch 6, wobei
a) das medizinische Produkt (1) einen Stent, einen Endograft oder eine Endoprothese umfasst, oder
b) das medizinische Produkt (1) einen Barestent und/oder einen Stentgraft umfasst.

8. Medizinisches Produkt zur Verwendung gemäß mindestens einem der vorhergehenden Ansprüche,
a) zusätzlich zum Medikament (3, 13) umfassend einen anti-proliferativen Wirkstoff, und/oder
b) zusätzlich zum Medikament (3, 13) umfassend einen retardierenden Hilfsstoff.

9. Medizinisches Produkt zur Verwendung gemäß mindestens einem der vorhergehenden Ansprüche, wobei das medizinische Produkt zum Nachfüllen bzw. Nachbeschichten mit dem Medikament (3, 13) vorgesehen ist.

10. Farnesyltransferaseinhibitor zur Verwendung in einem Verfahren zur Behandlung oder Prävention eines Aneurysmas.

11. Farnesyltransferaseinhibitor zur Verwendung gemäß Anspruch 10, wobei das Aneurysma wie in den Ansprüchen 2 bis 4 definiert ist.

12. Farnesyltransferaseinhibitor zur Verwendung gemäß einem der Ansprüche 10 oder 11, wobei der Farnesyltransferaseinhibitor in einem medizinischen Produkt (1, 11) gemäß mindestens einem der Ansprüche 1 bis 9 enthalten ist.

13. Farnesyltransferaseinhibitor zur Verwendung gemäß einem der Ansprüche 10 bis 12, wobei der Farnesyltransferaseinhibitor zur Nachbehandlung eines Aneurysmas verwendet wird.

14. Farnesyltransferaseinhibitor zur Verwendung gemäß mindestens einem der Ansprüche 10 bis 13, wobei das medizinische Produkt (1, 11) an der erkrankten Stelle platziert und/oder fixiert wird und der Farnesyltransferaseinhibitor an der erkrankten Stelle abgegeben wird.

15. Farnesyltransferaseinhibitor zur Verwendung gemäß mindestens einem der Ansprüche 10 bis 14, oder Medizinisches Produkt zur Verwendung gemäß mindestens einem der Ansprüche 1 bis 9, wobei der Farnesyltransferaseinhibitor R11577, SCH66336, FTI-277, GGTI-298, BMS-214664, L-778 oder L-123 ist.

## Claims

1. A medical product (1, 11) introducible into a blood vessel (2, 12) comprising a drug (3, 13) for the treatment or prevention of an aneurysm **characterized in that** said drug (3, 13) is a farnesyltransferase inhibitor, for use in the treatment or prevention of an aneurysm.

2. The medical product for use according to claim 1, wherein the aneurysm is caused by a degeneration of a vascular wall, in particular wherein said vascular wall is the aortic wall.

3. The medical product for use according to claim 1 or 2, wherein the aneurysm is an *Aneurysma verum,* an *Aneurysma spirum,* an *Aneurysma dissecans* or a hybrid thereof, in particular wherein said aneurysm is *Aneurysma verum.*

4. The medical product for use according to at least one of claims 1 to 3, wherein the aneurysm is an aneurysm at the aorta, at the *Arteria carotis,* at an artery of an upper or lower limb, the *Arteria subclavia,* a renal artery, an encephalic artery, at the *Arteria iliaca* or at the coronary artery.

5. The medical product for use according to at least one of the preceding claims, wherein the medical product (11) is an angioplasty balloon, with which the diseased area in the blood vessel (12) is treatable with the drug (13), farnesyltransferase inhibitor.

6. The medical product (1) for use according to at least one of the preceding claims, wherein the medical product (1) is implantable inside the blood vessel (2), wherein the drug (3), farnesyltransferase inhibitor, is administrable by means of the medical product (1) at the blood vessel (2).

7. The medical product (1) for use according to claim 6, wherein
a) the medical product (1) comprises a stent, an endograft or an endoprothesis, or
b) the medical product (1) comprises a barestent and/or a stentgraft.

8. The medical product for use according to at least one of the preceding claims,
a) comprising an anti-proliferative agent in addition to the drug (3, 13) and/or
b) comprising a retarding adjuvant in addition to the drug (3, 13).

9. The medical product for use according to at least one of the preceding claims, wherein said medical product is intended for refilling respectively recoating with the drug (3, 13).

10. Farnesyltransferase inhibitor for use in a method for the treatment or prevention of an aneurysm.

11. Farnesyltransferase inhibitor for use according to claim 10, wherein the aneurysm is defined as in claims 2 to 4.

12. Farnesyltransferase inhibitor for use according to one of claims 10 or 11, wherein said farnesyltransferase inhibitor is included in a medical product (1, 11) in accordance with at least one of claims 1 to 9.

13. Farnesyltransferase inhibitor for use according to one of claims 10 to 12, wherein said farnesyltransferase inhibitor is used for after-treatment of an aneurysm.

14. Farnesyltransferase inhibitor for use according to at least one of claims 10 to 13, wherein the medical product (1, 11) is placed and/or fixed at the diseased area and said farnesyltransferase inhibitor is delivered at the diseased area.

15. Farnesyltransferase inhibitor for use according to at least one of claims 10 to 14, or medical product for use according to at least one of claims 1 to 9, wherein the farnesyltransferase inhibitor is R11577, SCH66336, FTI-277, GGTI-298, BMS-214664, L-778 or L-123.

## Revendications

1. Produit médicinal (1, 11) pouvant être inséré dans un vaisseau sanguin (2, 12) qui présente un médicament (3, 13) pour le traitement ou la prévention d'un anévrisme, **caractérisé en ce que** le médicament (3, 13) est un inhibiteur de la farnésyltransférase, pour l'utilisation lors du traitement ou de la prévention d'un anévrisme.

2. Produit médicinal pour son utilisation selon la revendication 1, où l'anévrisme est entraîné par une dégénérescence d'une paroi vasculaire, en particulier où la paroi vasculaire est la paroi de l'aorte.

3. Produit médicinal pour son utilisation selon la revendication 1 ou 2, où l'anévrisme est un *Aneurysma verum,* un *Aneurysma spirum,* un *Aneurysma dissecans* ou une forme mixte de ceux-ci, en particulier où l'anévrisme est un *Aneurysma verum.*

4. Produit médicinal pour son utilisation selon au moins l'une des revendications 1 à 3, où l'anévrisme est un anévrisme de l'aorte, de l'artère carotide, d'une artère d'un membre supérieur ou inférieur, de l'artère sous-clavière, d'une artère rénale, d'une artère cérébrale, de l'artère iliaque ou d'un vaisseau coronaire.

5. Produit médicinal pour son utilisation selon au moins l'une des revendications précédentes, où le produit médicinal (11) est un ballonnet d'angioplastie avec lequel le site lésé du vaisseau sanguin (12) peut être traité au moyen du médicament (13), inhibiteur de la farnésyltransférase.

6. Produit médicinal (1) pour son utilisation selon au moins l'une des revendications précédentes, où le produit médicinal (1) peut être implanté dans le vaisseau sanguin (2), où, au moyen du produit médicinal (1), le médicament (3), inhibiteur de la farnésyltransférase, peut être appliqué sur le vaisseau sanguin (2).

7. Produit médicinal (1) pour son utilisation selon la revendication 6, où
a) le produit médicinal (1) comprend un stent, un endogreffon ou une endoprothèse, ou
b) le produit médicinal (1) comprend un stent nu et/ou un greffon de type stent.

8. Produit médicinal pour son utilisation selon au moins l'une des revendications précédentes,
a) comprenant, en plus du médicament (3, 13), une substance active antiproliférative, et/ou
b) comprenant, en plus du médicament (3, 13), un adjuvant retardateur.

9. Produit médicinal pour son utilisation selon au moins l'une des revendications précédentes, où le produit médicinal est prévu pour être chargé ultérieurement ou recouvert ultérieurement avec le médicament (3, 13).

10. Inhibiteur de la farnésyltransférase pour son utilisation dans un procédé de traitement ou de prévention d'un anévrisme.

11. Inhibiteur de la farnésyltransférase pour son utilisation selon la revendication 10, où l'anévrisme est tel que défini dans les revendications 2 à 4.

12. Inhibiteur de la farnésyltransférase pour son utilisation selon l'une des revendications 10 ou 11, où l'inhibiteur de la farnésyltransférase est contenu dans un produit médicinal (1, 11) selon au moins l'une des revendications 1 à 9.

13. Inhibiteur de la farnésyltransférase pour son utilisation selon l'une des revendications 10 à 12, où l'inhibiteur de la farnésyltransférase est utilisé pour le traitement ultérieur d'un anévrisme.

14. Inhibiteur de la farnésyltransférase pour son utilisation selon au moins l'une des revendications 10 à 13, où le produit médicinal (1, 11) est placé sur le site lésé et/ou est fixé et l'inhibiteur de la farnésyltransférase est diffusé sur le site lésé.

15. Inhibiteur de la farnésyltransférase pour son utilisation selon au moins l'une des revendications 10 à 14 ou produit médicinal pour son utilisation selon au moins l'une des revendications 1 à 9, où l'inhibiteur de la farnésyltransférase est R11577, SCH66336, FTI-277, GGTI-298, BMS-214664, L-778 ou L-123.
